# FASCICULE DE BREVET EUROPEEN

(11) **EP 3 004 890 B1**
(45) Date de publication et mention de la délivrance du brevet: **11.05.2022**
(21) Numéro de dépôt: 14727373.4
(22) Date de dépôt: 23.05.2014
(51) Int. Cl.: G01N 33/68

(54) **PROCÉDÉ POUR CARACTÉRISER PAR SPECTROMÉTRIE DE MASSE EN TANDEM UN ÉCHANTILLON BIOLOGIQUE**
VERFAHREN ZUR CHARAKTERISIERUNG EINER BIOLOGISCHEN PROBE DURCH TANDEM-MASSENSPEKTROMETRIE
METHOD FOR CHARACTERISING A BIOLOGICAL SAMPLE BY TANDEM MASS SPECTROMETRY

(30) Priorité: 24.05.2013 FR 1354692
(43) Date de publication de la demande: 13.04.2016
(73) Titulaire: Commissariat à l'Énergie Atomique et aux Énergies Alternatives, 75015 Paris (FR)
(72) Inventeur: ARMENGAUD, Jean, F-30400 Villeneuve-lez-Avignon (FR); PIBLE, Olivier, F-84370 Bedarrides (FR)
(74) Mandataire: Brevalex
(86) Numéro de dépôt international: PCT/EP2014/060715
(87) Numéro de publication internationale: WO 2014/187983

(56) Documents cités:
- WO-A1-2005/103069
- WO-A1-2010/063911
- WO-A2-2004/011638
- US-A1- 2004 053 830
- US-A1- 2004 161 825
- US-A1- 2005 147 627
- US-A1- 2010 255 527
- ANNARITA FARINA ET AL: "A Step Further in the Analysis of Human Bile Proteome", JOURNAL OF PROTEOME RESEARCH, vol. 10, no. 4, 11 février 2011 (2011-02-11), pages 2047-2063, XP055100270, ISSN: 1535-3893, DOI: 10.1021/pr200011b
- T. LI ET AL: "Proteomics Analysis Reveals Post-Translational Mechanisms for Cold-Induced Metabolic Changes in Arabidopsis", MOLECULAR PLANT, vol. 4, no. 2, 17 janvier 2011 (2011-01-17), pages 361-374, XP055100278, ISSN: 1674-2052, DOI: 10.1093/mp/ssq078
- Dakshinamurthy Rajalingam ET AL: "Trichloroacetic acid-induced protein precipitation involves the reversible association of a stable partially structured intermediate", Protein Science, vol. 18, no. 5, 1 May 2009 (2009-05-01), pages 980-993, XP055462538, US ISSN: 0961-8368, DOI: 10.1002/pro.108

## Description

### DOMAINE TECHNIQUE

La présente invention appartient au domaine technique des analyses protéomiques.

L'invention consiste en un procédé simple de préparation ou d'extraction de peptides à partir d'échantillons ou d'organismes vivants ou inactivés, en présence ou non de leurs nutriments ou/et de contaminants ou/et de protéines sécrétées par les organismes, à des fins d'identification par spectrométrie de masse.

Plus particulièrement, la présente invention propose un procédé permettant l'analyse d'un échantillon biologique par spectrométrie de masse en tandem, ledit procédé comprenant la préparation d'un culot protéique caractéristique dudit échantillon, la migration de ce matériel biologique protéique sur un gel d'électrophorèse en conditions dénaturantes suivie par une étape de protéolyse dans le gel moyennant quoi des peptides sont générés en vue d'une analyse par spectrométrie de masse en tandem.

### ÉTAT DE LA TECHNIQUE ANTÉRIEURE

L'identification ou/et la quantification d'organismes par spectrométrie de masse basée sur l'identification de protéines ou de peptides spécifiques ou non, ou de profilage de masses ou spectres est une technique largement utilisée. Une telle utilisation touche le domaine de la santé (identification d'agents pathogènes ou de toxines), le domaine agro-alimentaire (identification d'organismes génétiquement modifiés, identification de contaminants, validation de produits), le domaine de la surveillance environnementale (dosage d'organismes dans l'eau ou prélèvements aériens), le suivi de procédés industriels impliquant des organismes (chimie verte) ainsi que le typage de souches, d'organismes ou d'échantillons (taxonomie ou comparaison d'origine).

Les procédés actuels d'analyse en spectrométrie de masse nécessitent une préparation longue du matériel protéique pour générer les protéines ou les peptides à analyser. Typiquement, les cellules doivent être récoltées par centrifugation ou filtration, remises en suspension dans un tampon, puis lysées par des procédés chimiques ou physiques tels que sonication et broyage.

Par exemple, le brevet US 6,177,266 **[1]** propose un procédé pour générer, par spectrométrie de masse MALDI-TOF (pour « Matrix Assisted Laser Desorption/Ionization-Time Of Flight »), un profil de biomarqueurs spécifique d'une souche, d'une espèce ou d'un genre de bactéries. Ce profil peut être obtenu à partir d'extraits protéiques ou de cellules entières dans une solution aqueuse contenant de l'acide trifluoro-acétique (TFA), de l'acétonitrile et soit de l'acide α-cyano-4-hydroxycinnamique, soit de l'acide sinapique. Dans le procédé décrit dans le brevet US 6,177,266, les extraits protéiques sont préparés à partir d'un extrait cellulaire obtenu par sonication d'une solution contenant des cellules ayant préalablement été soumises à une lyophilisation. A noter que, lors de la préparation des extraits protéiques, une DNAse est utilisée pour en éliminer les acides nucléiques. La spectrométrie de masse MALDI-TOF, qui ne permet d'obtenir qu'un profil de biomarqueurs, peut être avantageusement remplacée par la spectrométrie de masse en tandem plus riche en informations discriminantes.

Le brevet US 6,558,946 **[2]** vise à améliorer la sensibilité et la résolution d'une analyse par spectrométrie utilisée pour préparer un profil de biomarqueurs. Cette amélioration est obtenue par une étape durant laquelle les biomarqueurs sont concentrés et séparés d'éventuels contaminants tels que solvants organiques, molécules ionisables, sels, enzymes et tensioactifs. Cette étape consiste en une ultrafiltration sur des (micro-)colonnes ou cartouches desquelles sont, par la suite, désorbés les biomarqueurs protéiques. Le procédé décrit dans le brevet US 6,558,946 peut aussi utiliser des biomarqueurs peptidiques, obtenus à partir des biomarqueurs protéiques précédemment obtenus mis en contact avec une cartouche imprégnée par une protéase.

La demande de brevet US 2010/255527 **[3]** propose un procédé pour identifier des pathogènes infectieux dans un fluide biologique. Ce procédé consiste à centrifuger le fluide biologique de façon à obtenir un 1^{er} culot dans lequel se trouvent les pathogènes infectieux, à soumettre le culot à une étape de décomposition et d'extraction en utilisant des acides et des solvants organiques et/ou des moyens physiques tels qu'un bain à ultrasons, à centrifuger la solution résultante et à soumettre le surnageant ainsi obtenu à une mesure par spectrométrie. Le procédé décrit ne concerne que les protéines internes dissoutes puisque le 1^{er} culot doit être lavé de façon à éliminer les protéines associées et les impuretés et que les protéines membranaires restent dans le 2^{nd} culot. Enfin, l'étape de décomposition/extraction est décrite comme participant à l'inactivation des pathogènes.

Farina et al. 2011 (J. Proteome Res., vol. 10, pages 2047-2063) propose un procédé pour caractériser par spectrométrie de masse en tandem un échantillon biologique correspondant à de la bile. Préalablement à cette caractérisation, ce procédé comprend une étape initiale de fractionnement dudit échantillon avec différentes centrifugations avant que le culot ainsi obtenu et resolubilisé en conditions dénaturantes ne soit soumis à une électrophorèse sur gel en conditions dénaturantes.

La demande de brevet US 2004/053830 concerne la protéine BCMP84 isolée d'une préparation membranaire d'une lignée cellulaire de cancer du sein. Le procédé pour isoler et identifier cette protéine consiste en une sonication des cellules, suivie d'une centrifugation, le culot contenant la partie membranaire étant, par la suite, soumis à une électrophorèse combinée à une spectrométrie de masse en tandem.

La demande internationale WO 2005/103069 vise à identifier et quantifier des protéines et des peptides de type biomarqueurs à partir d'échantillons complexes. Ces derniers sont clarifiés vi des étapes de centrifugation et d'ultrafiltration avant d'être

La demande de brevet US 2004/161825 décrit des protéines de surface spécifiques du sperme identifiées sur gel bidimensionnel et par microséquençage. Dans les exemples 1 et 3 mettant en œuvre une électrophorèse et une spectrométrie de masse, les échantillons sont soumis à une centrifugation.

La demande de brevet US 2010/255527 propose un procédé pour identifier par spectrométrie de masse du type MALDITOF des pathogènes infectieux dans les fluides biologiques. Dans ce procédé, une précipitation chimique est mise en œuvre sur un culot obtenu suite à une étape de précipitation.

La demande internationale WO 2004/011638 vise à fournir des polypeptides issus de *Streptococcus pneumonia* et *Enterococcus faecalis* et envisage que l'analyse des protéines soit réalisée par des techniques de spectrométrie de masse comme la spectrométrie de masse en tandem.

D'autres variantes de procédés pour détecter des micro-organismes dans un échantillon ou un fluide biologiques ont été décrites dans l'état de la technique. Ainsi, la demande internationale WO 2012/083150 **[4]** propose d'isoler et d'accumuler les micro-organismes par une étape de filtration qui suit une optionnelle étape de lyse. Cette demande internationale envisage également, lorsque l'échantillon contient des micro-organismes adhérents, de soumettre ce dernier à un prétraitement utilisant un tensioactif et/ou un vortex. Jabbour *et al* en 2011 **[5]** ont proposé l'utilisation d'un petit filtre afin de réaliser une séparation entre les peptides digérés des protéines entières, ce procédé nécessitant une lyse préalable des bactéries par sonication. Dans ce cas, les matières visqueuses présentes comme, par exemple, du glycérol souvent utilisé comme cryoconservant, peuvent obstruer le filtre. La présence de matrices complexes telles que des bactéries adhérentes à des graines ou fibres végétales peut rendre également ce procédé inefficace.

D'autre part, s'affranchir des inhibiteurs potentiels de la protéolyse ou d'agents qui peuvent atténuer les signaux en spectrométrie reste une étape problématique dans les procédés actuels et ne sont pas pris en compte ou sont passés sous silence. Par exemple, le protocole détaillé par Dworzanski *et al* en 2010 **[6]** afin de préparer des peptides à partir d'échantillons de bactéries à des fins d'identification comprend : un lavage des cellules par un tampon phosphate salin, une centrifugation, l'addition d'un réactif (« TRIO-Extraction reagent ») afin d'enlever les ADN et ARN, et une précipitation des protéines par l'isopropanol, suivi par un lavage du culot de protéines, puis dénaturation avec de la guanidine avant d'être traité par du DTT, alkylé à l'iodoacétamide, et digéré toute la nuit par de la trypsine. Jabbour *et al* en 2010 **[7]** ont proposé un protocole impliquant une filtration sur membrane et dénaturation toute la nuit à l'urée des protéines extraites.

De plus, dans le cas d'une identification d'organismes sous la forme d'un mélange dans l'échantillon, il est nécessaire d'avoir une méthode robuste permettant de produire des quantités relativement similaires de peptides entre organismes si une estimation quantitative doit être faite. Avec le protocole décrit par Jabbour *et al* **[5],** de fortes différences de rendement sont constatées entre bactéries de type Gram négatif et de type Gram positif (un facteur 5 a été rapporté).

D'autre part, une méthode permettant d'analyser les protéines ou leurs fragments produit(e)s par l'organisme et sécrété(e)s dans le milieu extérieur tel(le)s que toxines ou autres facteurs de virulence par exemple, ou des débris cellulaires provenant d'organismes ayant été mal conservés ou inactivés, peuvent également apporter des informations sur l'identité des organismes présents initialement dans l'échantillon. Les protocoles décrits à ce jour et notamment ceux décrits dans **[3-5]** ne permettent pas une telle analyse « tout en un ». Ces protéines et fragments seraient enlevés par le lavage ou la filtration des cellules.

Enfin, une méthode de préparation pour des identifications d'agents pathogènes ou susceptibles de l'être se doit d'être conduite après une étape d'inactivation drastique, en général traitement à la chaleur du type incubation 1 heure à 99°C ou traitement avec un acide fort, et être la plus simple possible afin d'éviter de multiplier les manipulations de l'échantillon. La préparation d'extraits protéiques à ce jour n'inclut pas cette étape et démarre par un matériel biologiquement actif ou ayant été conservé au froid.

Au vu de l'intérêt quant à l'obtention de procédés permettant l'identification ou/et la quantification d'organismes par spectrométrie de masse, les inventeurs se sont fixé pour but de proposer un procédé simple, pratique et qui ne présente pas les inconvénients des procédés listés ci-dessus.

### EXPOSÉ DE L'INVENTION

La présente invention permet de résoudre les inconvénients de l'état de la technique des procédés protéomiques pour identifier et éventuellement quantifier des organismes par spectrométrie de masse et d'atteindre le but que se sont fixé les inventeurs à savoir proposer un procédé simple de mise en œuvre et permettant une caractérisation des organismes contenus dans l'échantillon à partir de l'ensemble du contenu protéique de ce dernier sans risque de perdre une quelconque information. Plus précisément, l'invention consiste en un procédé simple de préparation de peptides à partir d'organismes ou d'extraits d'organismes, vivants ou inactivés, en présence ou non de leurs nutriments (milieu de culture) ou/et de contaminants ou/et de protéines sécrétées par les organismes, à des fins d'identification par spectrométrie de masse en tandem.

En effet, les travaux des inventeurs ont permis de mettre au point un procédé dans lequel l'échantillon n'est soumis à aucun traitement du type filtration, ultrafiltration ou lavage durant lequel une partie de l'information et notamment de l'information protéique contenue dans cet échantillon est perdue. De plus, le procédé selon l'invention ne nécessite pas l'utilisation de DNAses ou RNAses pour éliminer les acides nucléiques présents dans l'échantillon, ces derniers étant éliminés dans le surnageant obtenu suite à l'étape (b) ci-après définie et leurs éventuels résidus étant éliminés suite à l'étape (c) ci-après définie.

La mise en œuvre du procédé selon l'invention est rapide et pourrait être optimisée pour être réalisée en microcircuit. La rapidité de ce procédé et les conditions dénaturantes pour les protéines présentes ont également pour conséquence de ne pas nécessiter l'ajout d'inhibiteur de protéases lors de la préparation de l'échantillon. Les étapes (a), (b) et (c) ci-après définies sont réalisées dans un temps très court et en conditions dénaturantes minimisant de fait l'action des protéases sur l'échantillon.

Enfin, le procédé selon la présente invention est utilisable sur des échantillons de nature très variée et notamment sur des échantillons sur lesquels les procédés de l'art antérieur ne pouvaient être mis en œuvre. A titre d'exemples, les inventeurs ont utilisé ce procédé afin de préparer des échantillons de peptides à partir de bactéries pures d'origines diverses, en présence ou non du milieu de culture, de mélanges de bactéries, ou de bactéries adsorbées sur graines de fenugrec.

Plus particulièrement, la présente invention concerne un procédé tel que défini dans les revendications 1 à 7 pour caractériser par spectrométrie de masse en tandem un échantillon biologique choisi dans le groupe constitué par un fluide biologique ; un fluide végétal ; un prélèvement dans un milieu de culture ou dans un réacteur de culture biologique ; un liquide obtenu à partir d'un tissu animal ou végétal ; un tissu animal ou végétal ; une ou plusieurs cellules ; un prélèvement dans une matrice alimentaire ; un prélèvement dans un réacteur chimique ; un prélèvement dans une station d'épuration ; un prélèvement dans une station de compostage ; de l'eau de ville, de rivière, d'étang, de lac, de mer, de piscines, de tours aéro-réfrigérées ou d'origine souterraine; un prélèvement à partir d'un effluent industriel liquide ; de l'eau usée provenant notamment d'élevages intensifs ou d'industries du domaine chimique, pharmaceutique ou cosmétique ; un prélèvement provenant d'une filtration d'air ou d'un revêtement ; un prélèvement sur un objet : un produit pharmaceutique ; un produit cosmétique ; un parfum ; un échantillon de terre ou un de leurs mélanges, consistant à :
a) précipiter chimiquement du matériel biologique contenant des protéines, présent dans ledit échantillon en ajoutant un agent précipitant et dénaturant audit échantillon,
   ledit matériel biologique contenant des protéines étant choisi dans le groupe constitué par une cellule ; une partie de cellule ; un virus ; un prion et une protéine présente dans l'échantillon,
   ledit agent précipitant et dénaturant étant choisi dans le groupe constitué par un solvant organique, un composé inorganique acide ou basique, un composé organique acide, un sel chaotropique, une de leurs combinaisons ou un de leurs mélanges ;
b) soumettre ledit échantillon à une centrifugation moyennant quoi un culot comprenant le matériel biologique contenant des protéines est obtenu ;
c) soumettre le culot obtenu suite à l'étape (b) et resolubilisé en conditions dénaturantes à une électrophorèse sur gel en conditions dénaturantes ;
d) après d'éventuelles étapes préparatoires, soumettre tout ou partie des protéines du gel obtenu suite à l'étape (c) à une protéolyse et
e) soumettre les peptides obtenus suite à l'étape (d) à une spectrométrie de masse en tandem.

Le procédé selon la présente invention utilise la spectrométrie de masse (ou MS pour « Mass Spectrometry »). Cette dernière est une technique analytique déjà utilisée dans l'identification de mélanges complexes de composés chimiques et de molécules d'origine biologique et notamment en protéomique. Le principe de base d'une analyse par MS est la mesure du rapport masse/charge électrique d'espèces ioniques qui ont été créées par ionisation de l'échantillon à analyser et sur lesquelles sont appliqués des champs électriques et magnétiques.

La technique de MS plus particulièrement mise en œuvre, dans le cadre de la présente invention, est la spectrométrie de masse en tandem ou MS/MS. Dans cette technique, un ion stable particulier issu des espèces ioniques générées lors d'une première étape de MS (appelé « parent » ou « précurseur ») est sélectionné puis décomposé ou fragmenté moyennant quoi des ions issus de cette décomposition ou fragmentation (appelés « fils » ou « produits ») sont générés avant d'être analysés via une seconde étape de MS. Par une sélection fine des ions précurseurs (plage masse/charge électrique restreinte), seuls certains ions produits par certains analytes peuvent être introduits dans la chambre de fragmentation dans laquelle la collision avec des atomes d'un gaz inerte produit des ions fils. Comme les ions précurseurs tout comme les ions fils sont produits de façon reproductible pour des conditions d'ionisation/fragmentation données, la MS/MS fournit un outil analytique extrêmement intéressant notamment pour l'analyse d'échantillons complexes. Dernièrement, des méthodologies de MS/MS où les ions précurseurs sont sélectionnés sur une plage masse/charge électrique plus importante ont été proposées aboutissant à une identification des molécules par déconvolution des signaux d'ions fils complexes dérivant de plusieurs ions précurseurs.

Dans le cadre de la présente invention, la MS/MS est mise en œuvre sur des peptides obtenus selon le procédé revendiqué à partir de l'ensemble des protéines contenues initialement dans l'échantillon à caractériser. Cette MS/MS permet de générer des milliers de spectres de masse ou spectres MS/MS qui sont attribuables à des peptides, eux-mêmes pouvant être utilisés pour identifier les protéines présentes dans l'échantillon en utilisant des banques de données relatives aux protéines.

De plus, il est possible de quantifier une (ou plusieurs) protéine(s) dans l'échantillon de départ par différentes méthodes connues de l'homme du métier. A cet effet, des standards peuvent, par exemple, être soumis à une MS/MS en même temps que les peptides issus de l'échantillon et une courbe standard peut être construite à partir des signaux des ions générés à partir de ces standards. En utilisant cette courbe standard, l'abondance relative d'un ion donné peut être convertie en une quantité absolue de la protéine correspondante dans l'échantillon.

Par conséquent, par « procédé pour caractériser par spectrométrie de masse en tandem un échantillon », on entend, dans le cadre de la présente invention, aussi bien générer les spectres MS/MS caractéristiques des protéines contenues dans l'échantillon à analyser, qu'à partir de ces spectres, identifier et éventuellement quantifier les protéines contenues dans l'échantillon à analyser et, via ces protéines, identifier et éventuellement quantifier le (ou les) organisme(s) présent(s) dans ledit échantillon à analyser.

Il est évident que l'échantillon mis en œuvre dans le cadre du procédé selon la présente invention peut être de nature et de source très variées. Cet échantillon peut même être de nature inconnue comme un échantillon de type bioterrorisme ou un échantillon extraterrestre. De façon générale, il s'agit de tout échantillon pour lequel on souhaite caractériser par MS/MS le matériel biologique de nature protéique qu'il contient ou par lequel il est contaminé.

Cet échantillon est un fluide biologique ; un fluide végétal tel que sève, nectar et exsudat racinaire ; un prélèvement dans un milieu de culture ou dans un réacteur de culture biologique comme une culture cellulaire d'eucaryotes supérieurs, de levures, de champignons, de bactéries, de virus ou d'algues ; un liquide obtenu à partir d'un tissu animal ou végétal ; un tissu animal ou végétal ; une ou plusieurs cellules ; un prélèvement dans une matrice alimentaire ; un prélèvement dans un réacteur chimique ; un prélèvement dans une station d'épuration ; un prélèvement dans une station de compostage ; de l'eau de ville, de rivière, d'étang, de lac, de mer, de piscines, de tours aéro-réfrigérées ou d'origine souterraine; un prélèvement à partir d'un effluent industriel liquide ; de l'eau usée provenant notamment d'élevages intensifs ou d'industries du domaine chimique, pharmaceutique ou cosmétique ; un prélèvement provenant d'une filtration d'air ou d'un revêtement; un prélèvement sur un objet tel qu'un fragment de tissu, un habit, une semelle, une chaussure, un outil, une arme, etc... : un produit pharmaceutique ; un produit cosmétique ; un parfum ; un échantillon de terre ou un de leurs mélanges.

Dans le cadre de la présente invention, on entend par « prélèvement » tout type de collecte d'échantillons, par exemple, par contact, raclage, forage, découpage, poinçonnage, broyage, lavage, rinçage, aspiration, pompage, etc ...

Le fluide biologique est avantageusement choisi dans le groupe constitué par le sang tel que du sang entier ou du sang entier anti-coagulé, le sérum sanguin, le plasma sanguin, la lymphe, la salive, le crachat, les larmes, la sueur, le sperme, l'urine, les selles, le lait, le liquide céphalo-rachidien, le liquide interstitiel, un fluide isolé de moelle osseuse, un mucus ou fluide du tractus respiratoire, intestinal ou génito-urinaire, des extraits cellulaires, des extraits de tissus et des extraits d'organes. Ainsi, le fluide biologique peut être tout fluide naturellement sécrété ou excrété d'un corps humain ou animal ou tout fluide récupéré, à partir d'un corps humain ou animal, par toute technique connue de l'homme du métier telle qu'une extraction, un prélèvement ou un lavage. Les étapes de récupération et d'isolement de ces différents fluides à partir du corps humain ou animal sont réalisées préalablement à la mise en œuvre du procédé selon l'invention.

L'échantillon mis en œuvre dans le cadre de la présente invention peut contenir, de par sa nature ou éventuellement suite à une contamination, une (ou plusieur(s) cellule(s) (identiques ou différentes). Dans le cadre de la présente invention, on entend par « cellule », aussi bien une cellule de type procaryote que de type eucaryote. Parmi les cellules eucaryotes, la cellule peut être une levure telle qu'une levure du genre *Saccharomyces* ou *Candida,* une cellule végétale ou une cellule animale telle qu'une cellule de mammifères ou d'insectes. Les cellules de type procaryote sont des bactéries qui peuvent être de type gram positif ou de type gram négatif, ou des archées. Parmi ces bactéries, on peut citer, à titre d'exemples et de façon non-exhaustive, les bactéries appartenant aux embranchements des spirochètes et des chlamydiae, les bactéries appartenant aux familles des entérobactéries (telles que *Escherichia coli*)*,* des streptococcacées (telles que *Streptococcus*)*,* des microccacées (telles que *Staphylococcus*)*,* des légionelles, des mycobactéries, des bacillacées, des cyanobactéries et autres. Parmi ces archées, on peut citer, à titre d'exemples et de façon non-exhaustive, les archées appartenant aux phylas des Crenarchaeotes (telles que *Sulfolobus*) et Euryarchaeotes (telles que *Thermococcus*)*.*

L'échantillon mis en œuvre dans le cadre de la présente invention peut comprendre un (ou plusieurs) agent(s) pathogène(s). Dans ce cas, préalablement à la mise en œuvre du procédé selon l'invention, l'échantillon peut être soumis à un pré-traitement visant à l'inactiver. Toute technique couramment utilisée pour inactiver un échantillon comprenant des agents pathogènes est utilisable dans le cadre de ce pré-traitement. Avantageusement, ce pré-traitement peut consister à chauffer ledit échantillon à 99°C pendant une durée comprise entre 45 min et 90 min et, notamment, de l'ordre de 60 min (i.e. 60 min ± 10 min) puis à le laisser refroidir jusqu'à température ambiante (i.e. 22°C± 3°C). En variante, ce pré-traitement peut consister à chauffer ledit échantillon à une température supérieure à 99°C, notamment supérieure à 100°C et en particulier, de l'ordre de 110°C (i.e. 110°C± 5°C) pendant une durée comprise entre 5 min et 45 min et, notamment, de l'ordre de 20 min (i.e. 20 min ± 5 min) puis à le laisser refroidir jusqu'à température ambiante (i.e. 22°C± 3°C). Dans ce qui suit, on entend par « échantillon » aussi bien un échantillon ayant subi ce pré-traitement qu'un échantillon sans agent pathogène et, de fait, n'ayant pas subi une telle inactivation.

Lors de la mise en œuvre de l'étape (a) du procédé selon l'invention, l'échantillon se présente avantageusement sous forme liquide. Plus précisément, l'échantillon peut se présenter sous forme d'une solution, d'une suspension, d'une émulsion ou d'une micro-émulsion. Pour se présenter sous une telle forme, l'échantillon a pu subir une étape préalable de dilution. Ceci correspond notamment au cas où l'échantillon se présente initialement sous forme d'un tissu animal ou végétal ou d'un prélèvement dans une matrice alimentaire. Cette dilution peut se faire en ajoutant à l'échantillon, par exemple, de l'eau, un tampon salin ou un milieu de culture adapté aux cellules présentes dans l'échantillon.

L'étape (a) consiste à ajouter à l'échantillon un (ou plusieurs) agent(s) apte(s) à précipiter l'ensemble du matériel biologique contenant des protéines présent dans ledit échantillon. Cette étape (a), lorsque ledit échantillon comprend des cellules, entraîne la lyse de ces cellules.

Par « matériel biologique contenant des protéines », on entend, dans le cadre de la présente invention, une cellule notamment telle que précédemment définie; une partie de cellule; un virus; un prion et une protéine présente dans l'échantillon telle qu'une protéine présente dans la partie liquide de l'échantillon, une protéine sécrétée dans la partie liquide de l'échantillon comme une toxine ou une protéine présente dans la partie insoluble de l'échantillon comme, par exemple, dans le cas de biofilms.

Par « partie de cellule », on entend dans le cadre de la présente invention tout ou partie de la membrane cellulaire, tout ou partie des organites cellulaires tels que des noyaux, des mitochondries, l'appareil de Golgi, des endosomes ou des lysosomes et/ou tout ou partie des protéines présentes dans le milieu intracellulaire.

Par « membrane cellulaire », on entend dans le cadre de la présente invention aussi bien la membrane plasmique riche en phospholipides des cellules eucaryotes (également appelée membrane cytoplasmique, plasmalemme ou membrane plasmatique) que la membrane plasmique et la paroi cellulaire glucidique (contenant du peptidoglycane) des bactéries ou des cellules de plantes.

L'agent apte à précipiter un matériel biologique contenant des protéines est capable de précipiter l'ensemble du matériel biologique contenant des protéines contenu dans l'échantillon et cette précipitation s'accompagne d'une dénaturation irréversible des protéines. Ainsi, l'agent mis en œuvre lors de l'étape (a) est un agent précipitant et dénaturant. Avantageusement, cet agent est un agent précipitant, dénaturant et inactivant.

Ainsi, l'étape (a) du procédé selon l'invention consiste à ajouter à l'échantillon un élément choisi dans le groupe constitué par un solvant organique, un composé inorganique acide ou basique (un composé susceptible de modifier le pH de l'échantillon), un composé organique acide, un sel chaotropique, une de leurs combinaisons ou un de leurs mélanges. Avantageusement, l'étape (a) du procédé selon l'invention consiste à ajouter à l'échantillon un élément choisi dans le groupe constitué par un composé susceptible de modifier le pH de l'échantillon, un composé organique acide, un sel chaotropique, une de leurs combinaisons ou un de leurs mélanges.

Dans un mode de réalisation particulier, l'agent précipitant est un solvant organique et notamment de l'acétone, du chloroforme, un alcool tel que du méthanol ou de l'éthanol, ou un de leurs mélanges. A titre d'exemple, l'étape (a) du procédé selon l'invention peut consister à ajouter 4 ou 5 volumes d'acétone froid (-20°C) par volume d'échantillon, à mélanger l'ensemble au moyen d'un vortex et à laisser incuber le mélange obtenu à -20°C pendant une durée typiquement comprise entre 10 et 80 min. Un mélange de solvant organique utilisable lors de l'étape de précipitation du procédé selon l'invention est un mélange chloroforme/méthanol.

Dans un autre mode de réalisation particulier, l'agent précipitant est un composé inorganique acide ou basique i.e. un composé susceptible de modifier le pH de l'échantillon. Par « composé susceptible de modifier le pH de l'échantillon », on entend un composé typiquement inorganique qui, une fois ajouté à l'échantillon présentant initialement un pH désigné pHᵢ, entraîne une modification de ce pH, ce dernier présentant alors (a) soit une valeur supérieure à pHᵢ + 1, notamment supérieure à pHᵢ + 2, en particulier supérieure à pHᵢ + 3, et, plus particulièrement supérieure à pHᵢ + 4 ; (b) soit une valeur inférieure à pHᵢ - 1, notamment inférieure à pHᵢ - 2, en particulier inférieure à pHᵢ - 3, et, plus particulièrement inférieure à pHᵢ - 4 . En effet, il est connu que même des concentrations modérées d'acides ou de bases peuvent induire une précipitation des protéines. L'homme du métier connaît des composés inorganiques acides ou basiques utilisables pour modifier le pH de l'échantillon tels que l'acide chlorhydrique, l'acide perchlorique (PCA), l'hydroxyde de sodium ou l'hydroxyde de potassium et saura déterminer, sans effort inventif, la quantité à utiliser en fonction du pH initial de l'échantillon, du pH à atteindre et du (ou des) composé(s) modificateur(s) de pH utilisé(s).

Dans un autre mode de réalisation particulier, l'agent précipitant est un composé organique acide notamment choisi dans le groupe constitué par l'acide trichloroacétique (TCA), l'acide picrique ou l'acide sulfosalicylique. A titre d'exemple, l'étape (a) du procédé selon l'invention peut consister à ajouter du TCA à l'échantillon de façon à obtenir un échantillon à 10% (v/v) TCA, à mélanger au moyen d'un vortex et éventuellement à laisser incuber le mélange obtenu à 4°C pendant une durée typiquement comprise entre 5 et 30 min.

Dans un autre mode de réalisation encore, l'agent précipitant peut être un sel chaotropique, notamment choisi dans le groupe constitué par un sel de perchlorate, un sel de thiocyanate, un sel d'hydrogénosulfate, un sel de dihydrogénophosphate, un sel d'hydrogénocarbonate, un sel d'iodure, un sel de chlorure, un sel de nitrate, un sel de chlorure de guanidium, un sel d'urée et un sel de trichloroacétate. De plus amples informations sur ces sels et sur leur utilisation pour précipiter des protéines et, de façon plus générale, un matériel biologique contenant des protéines sont disponibles dans la demande internationale WO 03/076456 **[8].**

Une fois l'ensemble du matériel biologique contenant des protéines présent dans ledit échantillon précipité, l'échantillon est soumis à une centrifugation de façon à générer un culot contenant l'ensemble du matériel biologique contenant des protéines préalablement précipité. L'étape (b) de centrifugation du procédé selon l'invention est réalisée à haute vitesse, i.e. à une force centrifuge supérieure à 10000 g, notamment supérieure à 12000 g et, en particulier, comprise entre 13000 et 17000 g. Cette étape de centrifugation présente une durée comprise entre 1 et 15 min, notamment entre 2 et 10 min et, en particulier, de l'ordre de 5 min (i.e. 5 min ± 2 min).

Une fois l'étape (b) réalisée et préalablement à la mise en œuvre de l'étape (c), le surnageant est éliminé. Toute technique visant à éliminer un surnageant est utilisable dans le cadre de la présente invention. A titre d'exemples, cette élimination peut être réalisée par tapotement, par absorption ou par aspiration.

A noter que l'étape de centrifugation peut être répétée plusieurs fois de façon à débarrasser le culot comprenant le matériel biologique contenant des protéines, de toute solution piégée dans ledit culot ou présente de façon résiduelle sur les parois du contenant.

L'étape (c) du procédé selon la présente invention consiste à (c₁) reprendre (i.e. resolubiliser) le culot obtenu suite à l'étape (b) dans une solution dénaturante, (c₂) chauffer le mélange ainsi obtenu, (c₃) déposer le mélange obtenu suite à l'étape (c₂) sur un gel de polyacrylamide et (c₄) le soumettre à une migration en conditions dénaturantes. Les étapes (c₁) à (c₄) sont des étapes classiques dans le domaine de la biochimie. L'utilisation de l'électrophorèse en conditions dénaturantes permet de s'affranchir des solutés et métabolites présents dans l'échantillon obtenu suite à l'étape (b) qui peuvent inhiber la protéolyse mise en œuvre lors de l'étape (d) du procédé selon l'invention.

Il convient de noter que le culot obtenu suite à l'étape (b) n'est soumis, préalablement à l'étape (c), à aucun traitement visant à en sélectionner une partie qui est ultérieurement soumise à l'étape (c) du procédé selon l'invention. Un tel traitement peut consister en une chromatographie d'adsorption ou d'affinité.

La solution dénaturante mise en œuvre lors de l'étape (c₁) est une solution tampon comprenant un agent dénaturant du type détergent tel que du dodécyl sulfate de sodium (SDS) ou du dodécyl sulfate de lithium (LDS) ; du glycérol ; un agent réducteur tel que du béta-mercaptoéthanol ou du dithiothreitol (DTT) ; et éventuellement un colorant tel que du bleu de bromophénol, du rouge phénol, du bleu de Coomassie ou un de leurs mélanges. Le solvant de cette solution est typiquement choisi parmi le Tris, le Tris base, le Tris/HCl, la triéthanolamine ou un de leurs mélanges. Différentes solutions dénaturantes sont accessibles dans le commerce ou, en variante, l'homme du métier connaît plusieurs protocoles pour préparer une telle solution dénaturante. L'étape (c₁) peut nécessiter un mélange au vortex pour obtenir une remise en suspension suffisante du culot protéique et/ou une étape de sonication permettant de désagréger les particules présentes dans le culot protéique.

Lors de l'étape (c₂) du procédé selon l'invention, le mélange obtenu est chauffé à une température comprise entre 60 et 100°C et ce, pendant une durée comprise entre 2 et 15 min. Dans une forme de mise en œuvre particulière de l'étape (c₂) du procédé selon l'invention, le mélange obtenu peut être chauffé à une température de l'ordre de 95°C (i.e. 95°C ± 3°C) et ce, typiquement pendant une durée de l'ordre de 5 min (i.e. 5 min ± 2 min). Dans une autre forme de mise en œuvre particulière de l'étape (c₂) du procédé selon l'invention, le mélange obtenu peut être chauffé à une température de l'ordre de 70°C (i.e. 70°C ± 3°C) et ce, typiquement pendant une durée de l'ordre de 10 min (i.e. 10 min ± 2 min).

Le gel de polyacrylamide utilisable lors de l'étape (c₃) est une matrice polymère dans laquelle les protéines complexées au détergent de la solution dénaturante migrent en fonction de leur taille (gel de séparation ou de résolution). Ce gel peut être un gel du commerce ou un gel préalablement préparé par toute technique de biochimie connue de l'homme du métier. Un tel gel est typiquement préparé à partir d'acrylamide ; d'un agent réticulant comme, par exemple, de la bisacrylamide ou du diacrylamide de piperazine ; d'un détergent tel que précédemment défini ; d'un accélérateur de la polymérisation comme, par exemple, du N,N,N',N'-tétraméthylénediamine (TEMED) ou du 3-diméthylaminopropionitrile (DMNAPN) et d'un initiateur de la polymérisation tel que du persulfate d'ammonium. Ces différents éléments sont mélangés dans un tampon comprenant typiquement un (ou plusieurs) élément(s) choisi(s) entre du Tris, du Tris base, du Tris/HCl, de la triéthanolamine, de la glycine et un détergent tel que précédemment défini. Le gel mis en œuvre dans le cadre de l'étape (c₃) du procédé selon l'invention peut présenter un quelconque pourcentage en polyacrylamide, ce pourcentage pouvant être constant dans tout le gel ou varier sur la hauteur du gel (gradient). Avantageusement, le gel mis en œuvre présente un pourcentage de polyacrylamide compris entre 3 et 15% et notamment entre 4 et 12%. Un volume compris entre 1 et 40 µL et notamment entre 10 et 30 µL du mélange obtenu suite à l'étape (c₂) est déposé (chargé) par puits du gel de polyacrylamide. Une dilution de l'échantillon dans de la solution dénaturante peut être réalisée si la charge en protéines est trop importante.

L'homme du métier connaît différents tampons utilisables lors de la migration des protéines contenues dans l'échantillon, dans le gel de polyacrylamide, une fois le système mis sous tension. La valeur de la tension appliquée est comprise entre 100 et 250 V. La durée de l'électrophorèse peut être plus ou moins longue. Cette dernière peut être stoppée dès que l'échantillon est entré totalement dans le gel de résolution. Dans ce cas, l'électrophorèse peut durer entre 2 et 10 min et notamment de l'ordre de 5 min (i.e. 5 min ± 2 min). Alternativement, l'électrophorèse peut être prolongée afin de découper une bande de polyacrylamide contenant une fraction du protéome présent dans l'échantillon (de masse moléculaire faible ou élevée ou intermédiaire).

L'étape (d) consiste à préparer à partir de l'ensemble des protéines (i.e. le protéome) présentes dans le gel (électrophorèse courte) ou de certaines de ces protéines (électrophorèse prolongée) des peptides analysables par MS/MS.

Suite à l'électrophorèse en conditions dénaturantes et préalablement à cette étape de protéolyse, le procédé selon la présente invention peut présenter des étapes préparatoires consistant à :
d₁) colorer le gel obtenu suite à l'étape (c) notamment pour visualiser les protéines qu'il contient. L'homme du métier connaît différents protocoles, tampons et colorants utilisables à cet effet.
d₂) découper une partie de ce gel contenant tout ou partie des protéines initialement contenues dans l'échantillon. Cette découpe peut être réalisée de façon manuelle, par exemple, à l'aide d'une lame de rasoir ou d'un scalpel ou de façon automatisée par exemple à l'aide d'un emporte-pièce automatisé.
d₃) décolorer le gel découpé lors de l'étape (d₂). L'homme du métier connaît différents protocoles et tampons comprenant notamment du tampon bicarbonate utilisables pour cette décoloration. A noter que le gel découpé peut conserver une légère coloration sans que cette dernière n'affecte la performance du procédé de l'invention.
d₄) soumettre à une réduction et à une alkylation les protéines contenues dans le gel découpé et décoloré lors de l'étape (d₃). Cette étape (d₄) vise à dénaturer les protéines présentes dans le gel découpé en leur structure primaire et consiste plus particulièrement à couper les ponts disulfure qu'elles présentent (réduction) et à empêcher que ces derniers ne se reforment (alkylation). Tout agent réducteur est utilisable pour couper les ponts disulfures; avantageusement, il s'agit de béta-mercaptoéthanol ou de dithiothreitol (DTT). L'alkylation consiste à ajouter un groupement acétoamide sur les groupements sulfhydryle (-SH) issus de la coupure des ponts disulfures. Tout agent alkylant est utilisable à cet effet ; avantageusement, il s'agit d'iodoacétamide.

L'étape de protéolyse mise en œuvre dans le cadre de la présente invention est avantageusement une étape de protéolyse (ou digestion) enzymatique. Ainsi, cette étape consiste à mettre les protéines, et notamment les protéines réduites et alkylées, présentes dans le gel en présence avec au moins une protéase et avantageusement au moins une endoprotéase et ce pour conduire, de façon reproductible, à la formation de fragments peptidiques plus ou moins courts à partir des protéines contenues dans l'échantillon.

L'homme du métier connaît différentes protéases et notamment différentes endoprotéases utilisables lors de l'étape de cette protéolyse et les conditions opératoires à utiliser telles que tampon et température pour que ces enzymes rompent efficacement des liaisons peptidiques dans les protéines contenues dans l'échantillon.

Avantageusement, la (ou les) protéase(s) mise(s) en œuvre lors de l'étape de digestion enzymatique est(sont) choisie(s) dans le groupe constitué par la trypsine, la chymotrypsine, la thrombine, la protéase du VIH, l'élastase, le facteur Xa, une endopeptidase à thiol telle que la capaïne ou une caspase, une endopeptidase spécifique de la lysine (endoLysN), une endopeptidase spécifique de l'arginine (endoArgC) et une endopeptidase spécifique de l'acide glutamique (endoGluC). La protéase plus particulièrement mise en œuvre lors de l'étape de protéolyse enzymatique est la trypsine. Plus particulièrement encore, cette trypsine est utilisée en présence d'au moins un détergent ou d'une solution contenant un détergent notamment commercialisée sous le nom de solution ProteaseMax^{®}.

Préalablement à l'étape (e) du procédé selon la présente invention, les peptides obtenus suite à l'étape (d) de protéolyse doivent être récupérés ou extraits du gel de polyacrylamide (i.e. de la matrice polymère). A cet effet, un tampon d'extraction est utilisé. Ce dernier comprend un (ou plusieurs) élément(s) choisi(s) dans le groupe constitué par de l'acide trifluoroacétique, de l'acide formique et de l'acétonitrile.

Le procédé selon la présente invention peut comprendre en outre une étape additionnelle suite à l'étape (e). Cette étape additionnelle consiste, à partir des spectres MS/MS obtenus suite à l'étape (e), à identifier la (ou les) protéine(s) présente(s) dans l'échantillon et éventuellement le (ou les) organisme(s) présent(s) dans l'échantillon. Cette étape additionnelle peut nécessiter une comparaison avec des spectres MS/MS obtenus à partir de protéine(s) ou d'organisme(s) connu(e)(s).

D'autres caractéristiques et avantages de la présente invention apparaîtront encore à l'homme du métier à la lecture des exemples ci-dessous donnés à titre illustratif et non limitatif.

### EXPOSÉ DÉTAILLÉ DE MODES DE RÉALISATION PARTICULIERS

### Etape 1 : Echantillon initial.

L'échantillon (typiquement 1 mL) contient un mélange d'organismes divers et dilués (10^{E}6 cellules au total par exemple) dans un milieu de culture, ou une suspension de tissus cellulaires désagrégés. Eventuellement, un ou plusieurs de ces organismes sont pathogènes.

### Etape 2 optionnelle : Inactivation de l'échantillon.

Cette étape consiste à inactiver l'échantillon dans le cas où ce dernier serait pathogène ou contiendrait des agents pathogènes. Cette inactivation consiste à détruire les cellules en incubant l'échantillon pendant 1 heure à 99°C, puis à le laisser refroidir.

### Etape 3 : Concentration et extraction de la fraction protéique de l'échantillon.

Dans le cas où l'étape 2 optionnelle n'est pas mise en œuvre, les cellules de l'échantillon sont lysées et le contenu protéique de l'échantillon est précipité en ajoutant une solution de 50% TCA (acide trichloroacétique préparé dans de l'eau à 50% w/w, soit 10 g de TCA qsp 10 mL H₂O) à raison de 10% TCA final (soit un ajout de 250 µL de TCA à 50 % pour 1 mL d'échantillon). Le mélange est fortement agité au vortex et centrifugé à haute vitesse (5 min, centrifugation de type eppendorf, 16000 g). Le surnageant est éliminé et la centrifugation est répétée (10 secondes) pour enlever les quelques gouttes résiduelles si besoin.

A ce stade, le culot obtenu comprend les débris particulaires présents dans l'échantillon initial ainsi que les protéines présentes dans le matériel biologique et les protéines présentes en-dehors du matériel biologique telles que des protéines et toxines sécrétées et les protéines du milieu de culture. L'ensemble des protéines sera analysé par spectrométrie de masse.

### Etape 4 : Reprise du culot de protéines précipitées pour SDS-PAGE.

Un volume de 30 µL de bleu de charge pour SDS-PAGE est ajouté au culot obtenu à **l'étape 3.** Ce bleu de charge est typiquement une solution NuPAGE LDS Sample Buffer 1X (Invitrogen) consistant en 106 mM Tris/HCl, 141 mM Tris Base, 2% de dodécyl sulfate de lithium (LDS), 10% glycérol, 0,51 mM d'acide éthylène diamine tétra-acétique (EDTA), 0,22 mM Serva Blue G250, 0,175 mM Phenol Red, pH 8,5 auquel a été ajouté du β-mercaptoéthanol à 2% final. Le mélange obtenu est fortement agité au vortex et l'échantillon est soumis à une forte sonication au moyen d'un bac à ultrasons pendant 10 min ou d'un microsonicateur introduit dans le volume de l'échantillon pendant 1 min et ce, pour resolubiliser le culot. L'échantillon est ensuite chauffé pendant 5 min à 95°C.

### Etape 5 : SDS-PAGE.

L'échantillon est déposé sur un gel SDS-PAGE pour une électrophorèse en conditions dénaturantes. Les conditions plus particulièrement utilisées sont un dépôt de 20 µL d'échantillon, gel 4-12% Bis-Tris gradient 10-well NuPAGE (gel d'Invitrogen), migration en tampon MES 1X d'Invitrogen sous 200 V. Toutefois, la migration des protéines dans ce gel est stoppée dès que l'échantillon est entré totalement dans le gel de résolution et typiquement après 5 min de migration. Le gel est ensuite coloré brièvement au bleu de Coomassie (typiquement SimplyBlue SafeStain d'Invitrogen, 5 min), puis décoloré à l'eau au moyen de 3 lavages de 5 min minimum. La bande de protéines correspondant au protéome global de l'échantillon est découpée dans un volume minimal de 250 µL correspondant à un fragment de gel d'acrylamide de 6 mm x 6 mm x 7 mm, à l'aide d'un scalpel.

### Etape 6 : Préparation des peptides pour analyse par spectrométrie de masse.

La bande de gel de polyacrylamide est décolorée par 200 µL de mélange méthanol/bicarbonate d'ammonium (10 mL de méthanol mélangé à 10 mL d'ammonium bicarbonate à 50 mM). La bande recouverte du mélange est agitée pendant 1 min à 500 rpm, puis le liquide est enlevé. Cette étape de décoloration est répétée une fois, une légère coloration est toutefois tolérée.

La bande de gel de polyacrylamide est en partie déshydratée par 200 µL d'un mélange acétonitrile et bicarbonate d'ammonium. La bande recouverte de solution est agitée pendant 5 min à 600 rpm, puis le liquide est enlevé. La bande de gel de polyacrylamide est complètement déshydratée par ajout de 200 µL d'acétonitrile pur. La bande recouverte d'acétonitrile est agitée pendant 5 min à 600 rpm, puis le liquide est enlevé. La bande de polyacrylamide est séchée au SpeedVac pour 2 à 5 min.

La bande de gel de polyacrylamide est réhydratée par 100 µL d'une solution de réduction à base de dithiothreitol à 25 mM (38,5 mg de dithiothreitol dans 10 mL de bicarbonate d'ammonium à 500 mM) durant 20 min à 56°C, sous agitation à 500 rpm, puis le liquide est enlevé.

La bande de gel de polyacrylamide est mise en contact avec une solution d'alkylation. Pour cela, 100 µL d'iodoacétamide à 55 mM (102 mg d'iodoacétamide dans 50 mM de bicarbonate d'ammonium) sont ajoutés et laissés incuber pendant 20 min à température ambiante dans le noir; puis le liquide est enlevé. La bande de polyacrylamide est lavée par 400 µL d'eau déionisée pendant 1 min à 600 rpm, puis le liquide est enlevé. Cette étape de lavage est répétée une fois.

La bande de gel de polyacrylamide est déshydratée par 200 µL d'une solution acétonitrile:bicarbonate d'ammonium, sous agitation pendant 5 min à 600 rpm, puis le liquide est enlevé. La bande de gel de polyacrylamide est complètement déshydratée par ajout de 200 µL d'acétonitrile pur. La bande recouverte d'acétonitrile est agitée pendant 1 min à 600 rpm, puis le liquide est enlevé. La bande de polyacrylamide est séchée au SpeedVac pour 2 à 5 min.

La bande de polyacrylamide est réhydratée par 20 µL de solution enzymatique consistant en 2 µL de trypsine (type sequencing grade, Roche) reconstituée à 0,1 µg/µL dans 0,01% d'acide trifluoroacétique, 2 µL de ProteaseMax (Promega) à 0,1% et 16 µL de bicarbonate d'ammonium à 50 mM, et laissée incuber pendant 20 min sur la glace. L'excès de liquide est enlevé. Afin d'accroître l'action de la protéase et recueillir plus de peptides, il est possible d'ajouter 50 µL de solution ProteaseMax (Promega) à 0,01% puis de remuer brièvement. L'échantillon est incubé pendant 4 h à 37°C.

La solution est transférée dans un tube propre dans lequel sont ajoutés un volume d'acide trifluoroacétique pour ajuster la solution à 5% final. Eventuellement, dans le cas où moins de 50 µL de solution ont été obtenus, un volume, correspondant au complément à 50 µL, d'une solution à 0,1% d'acide trifluoroacétique est ajouté à la bande de polyacrylamide. La bande de polyacrylamide est agitée pendant 5 min sous 500 rpm avant de prélever le surnageant et l'ajouter à la solution prélevée précédemment.

La solution ainsi obtenue correspond à l'échantillon de peptides prêt pour être analysé en spectrométrie de masse, pour laquelle 1 à 10 µL de solution est(sont) utilisé(s). Dans le cas où un excès de protéines est présent dans l'échantillon, une dilution de cette solution de peptides est préparée.

### Etape 7 : Spectrométrie de masse et résultats.

Après la protéolyse, les peptides ont été injectés dans un spectromètre de masse en tandem XL LTQ-Orbitrap, couplé à une chaine nanoHPLC pour séparation des peptides par phase inverse, et opéré avec des paramètres standards connus de l'homme du métier. Les spectres MS/MS ont été analysés comme suit.

Pour chaque organisme, la base de données correspondante, sous format FASTA, a été construite par extraction à partir du site web du National Center for Biotechnology Information (NCBI) d'une liste des accessions de protéines (gi) correspondant à l'identificateur taxonomique NCBI de l'organisme. Un fichier contenant la liste des gi a été utilisé pour extraire un fichier FASTA non redondant en utilisant un outil « blast package tool » (blastdbcmd) avec la base de données blast NCBInr, en utilisant le paramètre « -entry_batch ». Une base de données « Mascot » a été créée à partir de ce fichier FASTA non redondant. Il a été utilisé pour traiter le fichier de données de spectrométrie de masse en tandem de type RAW obtenu pour le micro-organisme correspondant avec les paramètres standards du logiciel MASCOT (MatrixScience) (spécificité de la trypsine, le nombre maximal de défauts de coupure de la chaîne polypeptidique consécutifs fixé à 2), générant ainsi un fichier de type DAT. Le fichier DAT a été traité à l'aide d'un script python utilisant l'utilitaire d'analyse (msparser) de Mascot (Matrix Science) afin de recueillir le nombre de spectres MS/MS attribuables à des séquences peptidiques à une valeur de confiance p inférieure à 0,05. Ce nombre d'attributions, une fois divisé par le nombre total de MS/MS identifiés dans le fichier RAW, est un bon indicateur de l'efficacité d'extraction des peptides. Les pourcentages supérieurs à 50% dans ces conditions analytiques spécifiques sont des indicateurs clairs d'une bonne extraction des protéines et de bons rendements de la protéolyse des protéines.

Comme indiqué dans le tableau 1, les rendements d'extraction peptidique sont similaires (nombre élevé de spectres MS/MS enregistrés - plus de 1000 dans ces conditions - et attribution de spectres à des séquences peptidiques pour plus de 50% des spectres MS/MS enregistrés par le spectromètre de masse LTQ-Orbitrap XL tandem dans des conditions similaires) et ce, quelle que soit la coloration de Gram de la bactérie (*Escherichia coli* est une bactérie Gram-négative alors que *Bacillus cereus* est à Gram positif), ou si du glycérol (50% final) a été ajouté à la culture.

**Tableau 1**

| Organisme présent dans l'échantillon (nombre variable de cellules) | Complexité de l'échantillon ou réactifs additionnels | Tax id utilisé pour construire la base de données dédiée à partir de NCBInr | Nombre de spectres MS/MS enregistrés | Nombre de PSM assignés sur la base de données dédiée à une valeur p <0,05 | Pourcentage de spectres MS/MS assignés aux peptides de la base de données dédiée (%PSM) ^{∗} |
|---|---|---|---|---|---|
| *Escherichia coli BL21(DE3)* | Souche unique | 469008 | 6404^{b} | 4405^{b} | 69%^{b} |
| *Bacillus cereus ATCC 14579* | Souche unique | 226900 | 5311^{c} | 3474^{c} | 65%^{c} |
| *Francisella tularensis subsp. holarctica LVS* | Souche unique | 376619 | 2267 | 1433 | 63% |
| *Escherichia coli BL21(DE3)* | Souche unique en présence de 50% GSB^{a} | 469008 | 6159 | 4519 | 73% |
| *Escherichia coli BL21(DE3)* + *Ruegeria pomeroyi DSS-3* | Mélange de 2 organismes | 469008 + 246200 | 6317 | 2384 (469008) 2359 (246200) 4616 (469008 + 246200) | 73% (469008 + 246200) |

| | | | | | |
|---|---|---|---|---|---|
| ^{∗}Requête Mascot sur les bases de données dédicacées ; Peptide Spectrum Matches (PSM) filtrés à une valeur inférieure à 0,05 ^{a} GSB: Glycerol Strain Buffer (65% glycérol (v/v), 0,1 M MgSO₄, 0,025 M Tris-HCl pH 8) ^{b} Moyenne de trois replicats biologiques ^{e} Moyenne de deux replicats biologiques | | | | | |

### RÉFÉRENCES BIBLIOGRAPHIQUES

**[1]** Brevet US 6,177,266 au nom des Etats Unis d'Amérique représentés par le Secrétariat de l'Armée, publié le 23 janvier 2001.
**[2]** Brevet US 6,558,946 au nom des Etats Unis d'Amérique représentés par le Secrétariat de l'Armée et publié le 6 mai 2003.
**[3]** Demande de brevet US 2010/0255527 au nom de Weller, publiée le 7 octobre 2010.
**[4]** Demande internationale WO 2012/083150 au nom de Biomérieux, publiée le 21 juin 2012.
**[5]** Jabbour et al, 2011, « A protein processing filter method for bacterial identification by mass spectrometry based proteomics », J. Proteome Res., vol. 10, pages 907-912.
**[6]** Dworzanski et al, 2011, « Discrimination and phylogenic classification of Bacillus anthracis-cereus-thuringiensis strains based on LC-MS/MS analysis of whole cell protein digests », Anal. Chem., vol. 82, pages 145-155.
**[7]** Jabbour et al, 2010, « Identification of Yersinia pestis and Escherichia coli strains by whole cell and outer membrane protein extracts with mass spectrometry based proteomics », J. Proteome Res., vol. 9, pages 3647-3655.
**[8]** Demande internationale WO 03/076456 au nom de DSM IP ASSETS B.V., publiée le 18 septembre 2003.

## Revendications

1. Procédé pour caractériser par spectrométrie de masse en tandem un échantillon biologique choisi dans le groupe constitué par un fluide biologique ; un fluide végétal ; un prélèvement dans un milieu de culture ou dans un réacteur de culture biologique ; un liquide obtenu à partir d'un tissu animal ou végétal ; un tissu animal ou végétal; une ou plusieurs cellules; un prélèvement dans une matrice alimentaire; un prélèvement dans un réacteur chimique ; un prélèvement dans une station d'épuration ; un prélèvement dans une station de compostage ; de l'eau de ville, de rivière, d'étang, de lac, de mer, de piscines, de tours aéro-réfrigérées ou d'origine souterraine; un prélèvement à partir d'un effluent industriel liquide; de l'eau usée provenant notamment d'élevages intensifs ou d'industries du domaine chimique, pharmaceutique ou cosmétique ; un prélèvement provenant d'une filtration d'air ou d'un revêtement ; un prélèvement sur un objet ; un produit pharmaceutique ; un produit cosmétique ; un parfum ; un échantillon de terre ou un de leurs mélanges, consistant à :
a) précipiter chimiquement l'ensemble du matériel biologique contenant des protéines, présent dans ledit échantillon en ajoutant un agent précipitant et dénaturant audit échantillon,
ledit matériel biologique contenant des protéines étant choisi dans le groupe constitué par une cellule; une partie de cellule; un virus; un prion et une protéine présente dans l'échantillon,
ledit agent précipitant et dénaturant étant choisi dans le groupe constitué par un solvant organique, un composé inorganique acide ou basique, un composé organique acide, un sel chaotropique, une de leurs combinaisons ou un de leurs mélanges. ;
b) soumettre ledit échantillon à une centrifugation moyennant quoi un culot comprenant le matériel biologique contenant des protéines est obtenu ;
c) soumettre le culot obtenu suite à l'étape (b) et resolubilisé en conditions dénaturantes à une électrophorèse sur gel en conditions dénaturantes ;
d) après d'éventuelles étapes préparatoires, soumettre l'ensemble des protéines du gel obtenu suite à l'étape (c) à une protéolyse, et
e) soumettre les peptides obtenus suite à l'étape (d) à une spectrométrie de masse en tandem,
lesdites étapes préparatoires consistant à :
(d₁) colorer le gel obtenu suite à l'étape (c) ;
(d₂) découper une partie de ce gel contenant l'ensemble des protéines initialement contenues dans l'échantillon ;
(d₃) décolorer le gel découpé lors de l'étape (d₂) ;
(d₄) soumettre à une réduction et à une alkylation les protéines contenues dans le gel découpé et décoloré lors de l'étape (d₃).

2. Procédé selon la revendication 1, **caractérisé en ce que** préalablement à la mise en œuvre dudit procédé, l'échantillon est soumis à un pré-traitement visant à l'inactiver.

3. Procédé selon la revendication 1 ou 2, **caractérisé en ce que** ladite étape (a) consiste à ajouter audit échantillon un élément choisi dans le groupe constitué par un composé inorganique acide ou basique, un composé organique acide, un sel chaotropique, une de leurs combinaisons ou un de leurs mélanges.

4. Procédé selon la revendication 3, **caractérisé en ce que** ledit composé organique acide est choisi dans le groupe constitué par l'acide trichloroacétique (TCA), l'acide picrique ou l'acide sulfosalicylique.

5. Procédé selon l'une quelconque des revendications précédentes, **caractérisé en ce que** ladite étape (c) consiste à
(c₁) reprendre le culot obtenu suite à l'étape (b) dans une solution dénaturante,
(c₂) chauffer le mélange ainsi obtenu,
(c₃) déposer le mélange obtenu suite à l'étape (c₂) sur un gel de polyacrylamide et
(c₄) le soumettre à une migration en conditions dénaturantes.

6. Procédé selon l'une quelconque des revendications précédentes, **caractérisé en ce que** ladite étape de protéolyse est avantageusement une étape de protéolyse (ou digestion) enzymatique.

7. Procédé selon l'une quelconque des revendications précédentes, **caractérisé en ce que** ladite étape de protéolyse consiste à mettre les protéines présentes dans le gel en présence avec au moins une protéase choisie dans le groupe constitué par la trypsine, la chymotrypsine, la thrombine, la protéase du VIH, l'élastase, le facteur Xa, une endopeptidase à thiol telle que la capaïne ou une caspase, une endopeptidase spécifique de la lysine (endoLysN), une endopeptidase spécifique de l'arginine (endoArgC) et une endopeptidase spécifique de l'acide glutamique (endoGluC).

## Patentansprüche

1. Verfahren zur Charakterisierung einer biologischen Probe durch Tandem-Massenspektrometrie, ausgewählt aus der Gruppe bestehend aus einer biologischen Flüssigkeit; einer vegetarischen Flüssigkeit; einer Probenahme aus einem Kulturmilieu oder aus einem biologischen Kulturreaktor; einer aus einem tierischen oder vegetarischen Gewebe erhaltenen Flüssigkeit; einem tierischen oder vegetarischen Gewebe; einer oder mehreren Zellen; einer Probenahme aus einer Nahrungsmittelmatrix; einer Probenahme aus einem chemischen Reaktor; einer Probenahme aus einer Kläranlage; einer Probenahme aus einer Kompostieranlage; Wasser aus der Stadt, aus einem Fluss, aus einem Teich, aus einem See, aus dem Meer, aus Schwimmbädern, aus Luftkühltürmen oder unterirdischen Ursprungs; einer Probenahme aus einem Industrieabwasser; einem Abwasser insbesondere aus intensiver Tierhaltung oder Industrieanlagen im chemischen, pharmazeutischen oder kosmetischen Bereich; einer Probenahme aus einem Luftfiltersystem oder einer Beschichtung; einer Probenahme an einem Gegenstand; einem pharmazeutischen Produkt; einem kosmetischen Produkt; einem Parfüm; einer Bodenprobe oder einer ihrer Mischungen, wobei das Verfahren darin besteht:
a) das gesamte proteinhaltige biologische Material, das in der Probe vorliegt, chemisch auszufällen, indem der Probe ein Ausfällungs- und Denaturierungsmittel zugesetzt wird,
wobei das proteinhaltige biologische Material aus der Gruppe bestehend aus einer Zelle; einem Teil der Zelle; einem Virus; einem Prion und einem in der Probe vorliegenden Protein ausgewählt ist,
wobei das zum Ausfällungs- und Denaturierungsmittel aus der Gruppe bestehend aus einem organischen Lösungsmittel, einer sauren oder basischen anorganischen Verbindung, einer sauren organischen Verbindung, einem chaotropen Salz, einer ihrer Verbindungen oder einer ihrer Mischungen ausgewählt ist;
b) die Probe einer Zentrifugierung zu unterziehen, wodurch ein das proteinhaltige biologische Material enthaltendes Pellet erhalten wird;
c) das nach dem Schritt (b) erhaltene und unter denaturierenden Bedingungen wieder aufgelöste Pellet unter denaturierenden Bedingungen einer Gelelektrophorese zu unterziehen;
d) die gesamten, nach dem Schritt (c) erhaltenen Gelproteine nach möglichen Vorbereitungsschritten einer Proteolyse zu unterziehen, und
e) die nach dem Schritt (d) erhaltenen Peptide einer Tandem-Massenspektroskopie zu unterziehen,
wobei die Vorbereitungsschritte darin bestehen;
(d₁) das nach dem Schritt (c) erhaltene Gel zu färben;
(d₂) einen Teil dieses Gels, das die gesamten anfänglich in der Probe enthaltenen Proteine enthält, abzutrennen;
(d₃) das während des Schrittes (d₂) abgetrennte Gel zu entfärben;
(d₄) die in dem während des Schrittes (d₂) abgetrennten und entfärbten Gel enthaltenen Proteine einer Reduzierung sowie einer Alkylierung zu unterziehen.

2. Verfahren nach Anspruch 1, **dadurch gekennzeichnet, dass** die Probe vor der Durchführung des Verfahrens einer Vorbehandlung zur Inaktivierung derselben unterzogen wird.

3. Verfahren nach Anspruch 1 oder 2, **dadurch gekennzeichnet, dass** der Schritt (a) darin besteht, der Probe ein Element hinzuzufügen, das aus der Gruppe bestehend aus einer sauren oder basischen anorganischen Verbindung, einer sauren organischen Verbindung, einem chaotropen Salz, einer ihrer Kombinationen oder einer ihrer Mischungen ausgewählt ist.

4. Verfahren nach Anspruch 3, **dadurch gekennzeichnet, dass** die saure organische Verbindung aus der Gruppe bestehend aus Trichloressigsäure (TCA), Pikrinsäure oder Sulfosalicylsäure ausgewählt ist.

5. Verfahren nach einem der vorhergehenden Ansprüche, **dadurch gekennzeichnet, dass** der Schritt (c) darin besteht,
(c₁) das nach dem Schritt (b) erhaltene Pellet wieder in einer denaturierenden Lösung aufzunehmen,
(c₂) die derart erhaltene Mischung zu erhitzen,
(c3) die nach dem Schritt (c₂) erhaltene Mischung auf ein Polyacrylamid-Gel aufzubringen, sowie
(c4) diese einer Migration unter denaturierenden Bedingungen zu unterziehen.

6. Verfahren nach einem der vorhergehenden Ansprüche, **dadurch gekennzeichnet, dass** der Proteolyse-Schritt vorteilhafterweise ein enzymatischer Proteolyse- (oder Vergärungs-)Schritt ist.

7. Verfahren nach einem der vorhergehenden Ansprüche, **dadurch gekennzeichnet, dass** der Proteolyse-Schritt darin besteht, die in dem Gel vorhandenen Proteine mit mindestens einer Protease ausgewählt aus der Gruppe bestehend aus Trypsin, Chymotrypsin, Thrombin, VIH-Protease, Elastase, dem Faktor Xa, einer Thiol-Endopeptidase wie Calpain oder einer Caspase, einer spezifischen Lysin-Endopeptidase (endoLysN), einer spezifischen Arginin-Endopeptidase (endoArgC) sowie einer spezifischen Glutaminsäure-Endopeptidase (endoGluC) zusammenzubringen.

## Claims

1. A method for **characterising by** tandem mass spectrometry a biological sample selected from the group consisting of a biological fluid; a plant fluid; a sample from a culture medium or in a biological culture reactor; a liquid obtained from an animal or plant tissue; an animal or plant tissue; one or more cells; a sample from a food matrix; a sample from a chemical reactor; a sample from a sewage plant; a sample from a composting plant; city water, river water, pond water, lake water, sea water, swimming pool water, air-cooled tower water or underground water; a sample from a liquid industrial effluent; waste water originating in particular from intensive farming or industries in the chemical, pharmaceutical or cosmetics field; a sample from air filtration or coating; a sample from an object; a pharmaceutical product; a cosmetic product; a perfume ; a soil sample or one of the mixtures thereof, consisting in:
a) chemically precipitating all the biological material containing proteins, present in said sample by adding a precipitating and denaturing agent to said sample,
said biological material containing proteins being selected from the group consisting of a cell; a cell part; a virus; a prion and a protein present in the sample,
said precipitating and denaturing agent being selected from the group consisting of an organic solvent, an acid or base inorganic compound, an acid organic compound, a chaotropic salt, one of the combinations thereof or one of the mixtures thereof;
b) subjecting said sample to centrifugation whereby a pellet comprising the biological material containing proteins is obtained;
c) subjecting the pellet obtained after step (b) and resolubilised under denaturing conditions to gel electrophoresis under denaturing conditions;
d) after optional preparatory steps, subjecting all the proteins of the gel obtained after step (c) to proteolysis, and
e) subjecting the peptides obtained after step (d) to tandem mass spectrometry,
said preparatory steps consisting in:
(d₁) staining the gel obtained after step (c);
(d₂) cutting a part of this gel containing all the proteins initially contained in the sample;
(d₃) destaining the cut gel in step (d₂);
(d₄) subjecting the proteins contained in the gel cut and destained during step (d₃) to reduction and alkylation.

2. The method according to claim 1, **characterised in that** prior to the implementation of said method, the sample is subjected to a pre-treatment aiming at inactivating it.

3. The method according to claim 1 or 2, **characterised in that** said step (a) consists in adding to said sample an element selected from the group consisting of an acid or base inorganic compound, an acid organic compound, a chaotropic salt, one of the combinations thereof or one of the mixtures thereof.

4. The method according to claim 3, **characterised in that** said acid organic compound is selected from the group consisting of trichloroacetic acid (TCA), picric acid or sulfosalicylic acid.

5. The method according to any one of the preceding claims, **characterised in that** said step (c) consists in
(c₁) taking up the pellet obtained after step (b) in a denaturing solution,
(C₂) heating the mixture thus obtained,
(C₃) depositing the mixture obtained after step (C₂) on a polyacrylamide gel and
(C₄) subjecting it to migration under denaturing conditions.

6. The method according to any one of the preceding claims, **characterised in that** said proteolysis step is advantageously an enzymatic proteolysis (or digestion) step.

7. The method according to any one of the preceding claims, **characterised in that** said proteolysis step consists in contacting the proteins present in the gel with at least one protease selected from the group consisting of trypsin, chymotrypsin, thrombin, HIV protease, elastase, factor Xa, thiol endopeptidase such as capaine or caspase, lysine specific endopeptidase (endoLysN), arginine specific endopeptidase (endoArgC) and glutamic acid specific endopeptidase (endoGluC).
